(19) **Europäisches Patentamt**

**European Patent Office**

**Office européen des brevets**

(11) **EP 1 454 540 B1**

(12) **EUROPÄISCHE PATENTSCHRIFT**

(45) Veröffentlichungstag und Bekanntmachung des Hinweises auf die Patenterteilung:
**21.06.2006 Patentblatt 2006/25**

(51) Int Cl.:
*A23L 3/00* (2006.01)          *A61L 2/24* (2006.01)
*A61L 2/04* (2006.01)          *G05B 11/32* (2006.01)

(21) Anmeldenummer: **04005313.4**

(22) Anmeldetag: **05.03.2004**

(54) **Vorrichtung und Verfahren zur Pasteurisierung von Produkten**

Apparatus and process for pasteurising of products

Appareil et procédé de pasteurisation de produits

(84) Benannte Vertragsstaaten:
**AT BE BG CH CY CZ DE DK EE ES FI FR GB GR HU IE IT LI LU MC NL PL PT RO SE SI SK TR**

(30) Priorität: **07.03.2003 DE 10310047**

(43) Veröffentlichungstag der Anmeldung:
**08.09.2004 Patentblatt 2004/37**

(73) Patentinhaber: **SANDER HANSEN A/S**
**2605 Brondby (DK)**

(72) Erfinder: **Hansen, Lars Henrik**
**2605 Brondby (DK)**

(74) Vertreter: **Grünecker, Kinkeldey,**
**Stockmair & Schwanhäusser**
**Anwaltssozietät**
**Maximilianstrasse 58**
**80538 München (DE)**

(56) Entgegenhaltungen:
**CA-A- 1 080 830          DE-A- 3 637 661**
**US-A- 4 639 854          US-A- 5 360 594**

**Beschreibung**

Die Erfindung betrifft eine Vorrichtung zur Pasteurisierung von Produkten, insbesondere befüllten Behältern, wie Flaschen und Dosen, mit einer Regeleinrichtung, die einen ersten Regelkreis umfasst, der nach einem ersten Kriterium regelbar ist. Die Erfindung betrifft ferner ein Verfahren zum Pasteurisieren von Produkten, insbesondere von befüllten Behältern, wie Flaschen und Dosen, wobei ein erster Regelkreis nach einem ersten Kriterium geregelt wird.

Aus der US-A-5 360 594 ist ein Regelsystem für eine Entsorgungseinheit für medizinischen Abfall bekannt. In dieser Regeleinheit findet die Dekontaminierung mit Hilfe von zwei Regelkreisen statt, wobei diese in Form einer Kaskadenregelung überlagert sind.

In herkömmlichen Anlagen zur Wärmebehandlung, insbesondere zur Pasteurisierung von Produkten erfolgt die Regelung nach der Anzahl der übertragenen Pasteurisierungseinheiten (PE-Regelung). Die Berechnung der nachfolgend mit PE abgekürzten Pasteurisierungseinheiten erfolgt nach folgender Formel:

$$\frac{dPE}{dt} = 10^{\left(\frac{T_p - T_{ref}}{z}\right)}$$

In dieser Formel bezeichnet

$T_p$      die Produkttemperatur, wobei üblicherweise als Pasteurisierungsparameter

$T_{ref}$      = 60 °C und

z      gleich 6,949468.

In Abhängigkeit vom jeweiligen Produkt wird ein bestimmter Pasteurisierungsgrad festgelegt, der für eine ausreichende Haltbarmachung ohne Beeinträchtigung des Produktes erforderlich. Nach diesem Pasteurisierungsgrad bestimmen sich die vom jeweiligen Produkt aufzunehmenden Pasteurisierungseinheiten, deren Übertragung durch eine geeignete Temperaturregelung der Anlage gewährleistet sein muss.

Eine von der Anmelderin hergestellte Vorrichtung zur Pasteurisierung von Produkten ist aus dem europäischen Patent 0 437 499 B1 bekannt. Mit dieser bekannten Vorrichtung wird eine Über- bzw. Unterpasteurisierung der Produkte beim Anhalten der Anlage während der Wärmebehandlung vermieden, wobei der Energieverbrauch gleichzeitig gesenkt wird. Dies wird bei der bekannten Vorrichtung dadurch erreicht, dass abfließende Sprühflüssigkeit aus Zonen mit unterschiedlichen Temperaturen kreuzweise derart umgeleitet werden, dass kühlere Produkte in einem Pasteurisierungsgebiet dazu verwendet werden, um damit wärme Produkte an anderer Stelle in der Vorrichtung durch einen Wärmeausgleich der Sprühflüssigkeit in diesem Gebiet zu kühlen. Dadurch werden eine zusätzliche Zufuhr von kalter Sprühflüssigkeit oder ein Ablassen von warmer Sprühflüssigkeit und die damit verbundenen Energieverluste vermieden.

Die Regelung dieser bekannten Vorrichtung erfolgt ausschließlich nach der Anzahl der übertragenen Pasteurisierungseinheiten, die einen bestimmten Soll-Wert, d.h. einen bestimmten Pasteurisierungsgrad innerhalb enger Toleranzen, erreichen müssen.

DE 36 37 661 C2 offenbart ein Verfahren zum Pasteurisieren von Produkten, mit dessen Hilfe ein energiesparendes und sicheres Pasteurisieren auch dann ermöglicht werden soll, wenn Störungen beim Durchlauf der zu pasteurisierenden Behälter auftreten. Dazu wird eine Vorpasteurisierzone und eine Pasteurisierzone in regelbare Elementarzonen unterteilt, wobei in jeder Elementarzone wenigstens ein Referenzbehälter beobachtet wird unter Berechnung der Anzahl von Pasteurisierungseinheiten, die dieser Behälter aufnimmt. Bei Überschreiten oder Unterschreiten eines bestimmten Soll-Wertes wird das Abkühlen oder Erwärmen der jeweiligen Zone eingeleitet, wodurch bei Kenntnis der Durchlaufgeschwindigkeit eine optimale, variable Anpassung der Sprühtemperatur erfolgen kann.

Auch bei diesen bekannten Verfahren erfolgt die Prozessregelung ausschließlich nach der Anzahl der übertragenen Pasteurisierungseinheiten.

Eine Vorrichtung der eingangs genannten Gattung wird von der Anmelderin hergestellt und auf den Markt gebracht.

Die Regeleinrichtung dieser bekannten Vorrichtung unterstützt, wie auch die vorstehend beschriebene Anlage bzw. das vorstehend beschriebene Verfahren, die Prozessregelung nach einem Soll-Wert einer Regelgröße, insbesondere nach einem Soll-Wert der Pasteurisierungseinheiten.

Daneben gestattet die bekannte Vorrichtung auch die Überwachung eines oder mehrerer anderer Kriterien, beispielsweise der Abtöttemperatur, also der Temperatur, die reicht werden muss, damit eine Abtötung von Keimen erfolgt. Aufgrund des Überwachungsergebnisses wird gegebenenfalls eine Wärmebehandlung an den normalen Pasteurisierungsprozess angeschlossen, wenn beispielsweise ein Produkt eine bestimmte Temperaturhöhe, nämlich den Abtötpunkt, nicht erreicht, bevor es die letzte Wärmebehandlungszone verlässt. Ein das Produkt transportierendes Förderband wird dann angehalten, wobei die Wärmenachbehandlung durchgeführt wird, bis das überwachte Kriterium erfüllt ist, was beträchtliche Stillstandszeiten der Anlage zur Folge hat.

Bei diesem Eingriff in den Prozessablauf handelt es sich um eine Steuerung, d.h., es tritt kein in sich geschlossener Signalkreis auf, bei dem eine gesteuerte Ausgangsgröße auf eine steuernde Eingangsgröße zurückwirkt. Im Gegensatz zu einer Regelung liegt bei der bekannten Vorrichtung ein offener Wirkungsablauf vor, bei dem eine Angleichung einer Ist-Größe an eine Soll-Größe nicht stattfindet.

Insgesamt erfolgt die Regelung der bekannten Pasteurisieranlagen bisher nur nach einem einzigen Soll-Kriterium, meist dem Pasteurisierungsgrad. Außerdem ist es bekannt, zusätzlich zu der Pasteurisierungseinheiten-Regelung andere Kriterien, beispielsweise die Killing-Point-Temperature, zu überwachen, wobei aber das Überwachungsergebnis nicht in die Regelung mit einfließt, sondern nur dazu verwendet wird, bei Bedarf eine nachträgliche Wärmebehandlung anzuschließen.

Wenn also bei den bekannten Regelungen ein anderes Kriterium als das Regelkriterium im normalen Pasteurisierungsablauf nicht erfüllt ist, muss eine Nachbehandlung durchgeführt werden, bis das entsprechende Kriterium erfüllt ist. Dies zieht unerwünschte Stillstandszeiten der Anlage nach sich.

Die Aufgabe der Erfindung besteht demnach darin, eine Vorrichtung bzw. ein Verfahren zum Pasteurisieren zu schaffen, wobei längere Stillstandszeiten vermieden werden können, auch wenn ein anderes Kriterium als das Regelkriterium nicht erfüllt ist.

Diese Aufgabe wird bei der Vorrichtung zur Pasteurisierung von Produkten erfindungsgemäß durch den Gegenstand des Patentanspruchs 1 gelöst. Bei dem Verfahren zum Pasteurisieren von Produkten wird diese Aufgabe erfindungsgemäß durch den Gegenstand des Patentanspruchs 10 gelöst.

Die Erfindung bringt den Vorteil, dass mehrere unterschiedliche Kriterien gleichzeitig bei der Regelung berücksichtigt werden, wodurch die Güte der Regelung verbessert wird. Durch die gleichzeitige Berücksichtigung mehrerer Kriterien wird ferner die Zuverlässigkeit der Pasteurisierung erhöht, da dadurch verschiedene Sicherheitsaspekte, beispielsweise das Erreichen einer bestimmten Abtöttemperatur in die Regelung einfließen.

Außerdem wird bei der erfindungsgemäßen Vorrichtung während des normalen Prozessablaufs die Temperatur nachgeregelt, wenn absehbar ist, dass ein weiteres Kriterium nicht erfüllt wird. Auf diese Weise werden Stillstandszeiten verringert, verglichen mit einer bekannten Vorrichtung, die nach nur einem Kriterium geregelt wird und andere Kriterien lediglich überwacht und zur Steuerung verwendet.

Erfindungsgemäß ist der zweite Regelkreis mit dem ersten Regelkreis an einer Mischstelle verknüpft, wobei dieser Mischstelle ein Eingangssignal, das einem Soll-Wert des ersten Kriteriums entspricht, und ein Ausgangssignals des zweiten Regelkreises zuführbar sind. Das Ausgangssignal des zweiten Regelkreises moduliert das Eingangssignal des ersten Regelkreises. Diese Ausgestaltung der erfindungsgemäßen Vorrichtung setzt auf einfache Weise die Überlagerung des ersten Regelkreises mit dem zweiten Regelkreis um, wobei durch die Modulation des Eingangssignals des ersten Regelkreises durch das Ausgangssignal des zweiten Regelkreises eine Beeinflussung des Soll-Wertes des ersten Kriteriums erfolgt. Dadurch fließt die Regelung nach dem zweiten Kriterium des zweiten Regelkreises in den ersten Regelkreis ein.

In einer bevorzugten Ausgestaltung der Erfindung umfasst der erste Regelkreis einen Vergleicher, der der Mischstelle nachgeordnet ist, wobei der Vergleicher das in der Mischstelle modulierte Eingangssignal und ein weiteres Signal vergleicht, das einem Ist-Wert des ersten Kriteriums entspricht, und ein Ausgangssignal ausgibt, das einer sich aus dem Soll/Ist-Vergleich ergebenden Regelabweichung entspricht. Auf diese Weise wird das in der Mischstelle durch das Ausgangssignal des zweiten Regelkreise modulierte Eingangssignal des ersten Regelkreises als Führungsgröße verwendet, an die der Ist-Wert des ersten Kriteriums angepasst wird.

In einer weiteren vorteilhaften Ausgestaltung der erfindungsgemäßen Vorrichtung ist dem Vergleicher wenigstens ein Regler und Stellglied nachgeordnet, wobei das der Regelabweichung entsprechende Ausgangssignal des Vergleichers dem Regler zuführbar ist.

Der zweite Regelkreis weist bevorzugt einen Vergleicher auf, der ein Eingangssignal, das einem Soll-Wert des zweiten Kriteriums entspricht, und ein weiteres Signal vergleicht, das einem Ist-Wert des zweiten Kriteriums entspricht. Der Vergleicher gibt ein Ausgangssignal aus, das einer sich aus dem Soll/Ist-Vergleich ergebenden Regelabweichung entspricht. Durch diesen Soll/Ist-Vergleich geht vorteilhafterweise das zweite Regelkriterium in den zweiten Regelkreis ein, wodurch die Möglichkeit geschaffen wird, durch die Überlagerung des ersten Regelkreises mit dem zweiten Regelkreis dieses zweite Regelkriterium zur Modulation des Eingangssignals des ersten Regelkreises einzusetzen.

In einer weiteren vorteilhaften Ausgestaltung der erfindungsgemäßen Vorrichtung ist dem Vergleicher des zweiten Regelkreises ein Regler nachgeordnet, wobei das der Regelabweichung entsprechende Ausgangssignal des Vergleichers des zweiten Regelkreises diesem Regler zuführbar ist, der dasjenige Ausgangssignal ausgibt, das der Mischstelle zur Modulation des Eingangssignals des ersten Regelkreises zuführbar ist. Vorteilhafterweise erfolgt durch den Regler des zweiten Regelkreises eine Anpassung des Ist-Wertes des zweiten Kriteriums an den in den Vergleicher eingegebenen Soll-Wert des zweiten Regelkriteriums.

In einer weiteren vorteilhaften Ausgestaltung der erfindungsgemäßen Vorrichtung ist vorgesehen, dass der erste und der zweite Regelkreis jeweils eine Einrichtung zur Berechnung der Ist-Werte der jeweiligen Kriterien aus einem Prozessparameter, insbesondere der Spritztemperatur, umfassen. Der Vorteil dieser Ausgestaltung der Erfindung liegt darin, dass, ausgehend von einem einzigen Prozessparameter, insbesondere von der Spritztemperatur, unterschiedliche Regelkriterien bzw. deren Ist-Werte berechnet werden, so dass durch die Bestimmung verschiedener Kriterien verschiedene Aspekte des Prozessablaufes bei der Regelung berücksichtigt werden können.

Vorzugsweise umfasst das erste Kriterium Pasteurisiereinheiten und das zweite Kriterium eine andere Größe, wie beispielsweise eine Abtöttemperatur. Dadurch wird vorteilhafterweise bei Überlagerung des ersten Regelkreises, der nach Pasteurisiereinheiten geregelt wird, mit dem zweiten Regelkreis, der nach der Abtöttemperatur geregelt wird, sichergestellt, dass nicht nur ein ausreichender Pasteurisierungsgrad eingehalten wird, sondern dass auch die Abtöttemperatur beim Pasteurisieren nicht unterschritten wird, wodurch die Prozesssicherheit, d.h. das zuverlässige Abtöten von Keimen, verbessert wird. Wenn für das zweite Kriterium eine andere Größe gewählt wird, können entsprechend andere Schwerpunkte in die Regelung einfließen. Es kommt also darauf an, dass das erste und zweite Kriterium unterschiedlich sind, um verschiedene Regelungsaspekte berücksichtigen zu können.

In einer weiteren vorteilhaften Ausgestaltung der erfindungsgemäßen Vorrichtung können weitere Regelkreise vorgesehen sein, die jeweils nach weiteren Kriterien regelbar sind, wobei die Ausgangssignale der weiteren Regelkreise der Mischstelle zuführbar sind und das Eingangssignal des ersten Regelkreises beeinflussen. Durch diese Erweiterung der Regeleinrichtung ist eine noch komplexere und umfassendere Regelung des Prozessablaufes möglich, bei welchem noch weitere Kriterien berücksichtigt werden und in die Regelung eingehen.

Die weiteren Regelkreise können jeweils eine Mischstelle umfassen, an der jeder weitere Regelkreis mit dem vorangegangenen Regelkreis verknüpft ist. Auf diese Weise wird das Ausgangssignals des jeweils vorangegangenen Regelkreises durch das Ausgangssignal des weiteren Regelkreises moduliert, so dass das modulierte Ausgangssignal an die Mischstelle des ersten Regelkreises weitergeleitet werden kann, wodurch über das modulierte Gesamtausgangssignal der weiteren Regelkreise das Eingangssignal des ersten Regelkreises beeinflusst wird.

Die Erfindung wird im Folgenden anhand schematischer Zeichnungen beispielsweise und mit weiteren Einzelheiten erläutert. Dabei zeigen:

Fig. 1    ein Blockschaltbild mit Signalfluss eines Ausführungsbeispiels einer Regelung der erfindungsgemäßen Vorrichtung für N-Regelkreise und

Fig. 2    ein Blockschaltbild mit Signalfluss eines Ausführungsbeispiels einer Regelung der erfindungsgemäßen Vorrichtung mit zwei konkret ausgeführten Regelkreisen.

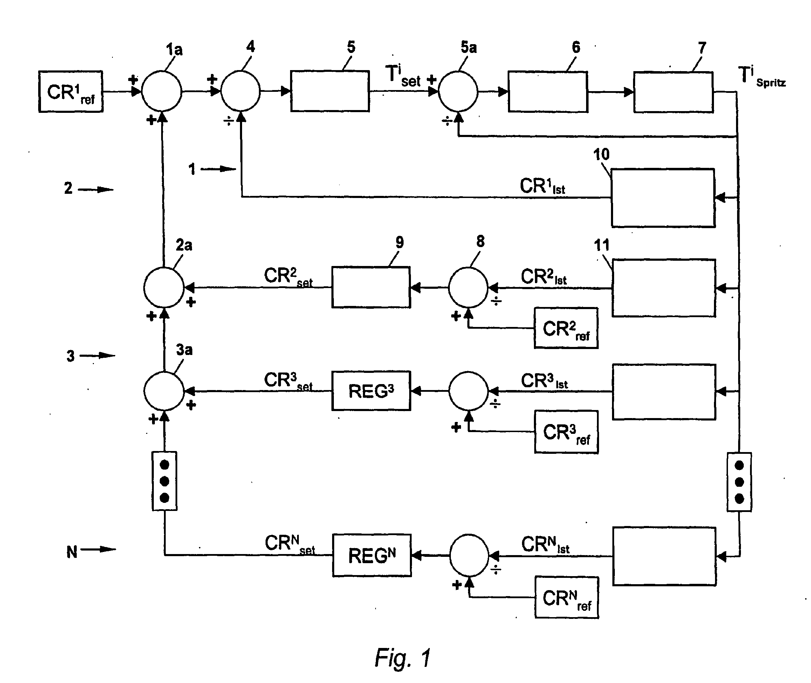

Fig. 1 zeigt in Form eines Blockschaltbildes die Regelung eines Ausführungsbeispiels der erfindungsgemäßen Vorrichtung. Diese Regelung wird von einer Regeleinrichtung ausgeführt, die einen Messfühler, Messumformer, Messverstärker, Soll-Wert-Geber, Vergleicher, Regler, Stellantrieb, Stellglied sowie andere Bauteile umfassen kann, von denen in Fig. 1 nur der Vergleicher, der Regler und das Stellglied dargestellt sind.

Selbstverständlich ist die Erfindung nicht auf die in Fig. 1 dargestellten Bestandteile der Regeleinrichtung beschränkt, sondern umfasst auch die vorstehend genannten weiteren Komponenten der Regeleinrichtung.

Wie in Fig. 1 gezeigt, ist dem ersten Regelkreis 1 wenigstens ein zweiter Regelkreis 2 überlagert, wobei, wie weiter in Fig. 1 zu erkennen, dem ersten Regelkreis 1 noch weitere Regelkreise 3 bis N überlagert sind.

Der erste Regelkreis 1 setzt sich wie folgt zusammen:

Nach dem in Fig. 1 gezeigten Signalflussplan des ersten Regelkreises wird ein Eingangssignal $CR^1_{ref}$, das einen Soll-Wert des ersten Regelkriteriums entspricht, über eine Mischstelle 1a einem Vergleicher 4 zugeführt wird, der das Eingangssignal $CR^1_{ref}$ mit einem weiteren Signal $CR^1_{ist}$ vergleicht, das einem Ist-Wert des ersten Kriteriums entspricht. Der Vergleicher 4 bestimmt eine Regelabweichung zwischen dem Soll-Wert und dem Ist-Wert des ersten Kriteriums und gibt ein in Fig. 1 nicht näher bezeichnetes Ausgangssignal aus, das dieser Regelabweichung entspricht. Dieses Ausgangssignals des Vergleichers 4 wird einem Regler 5 des ersten Regelkreises 1 zugeführt. Dieser Regler 5 kann beispielsweise ein PI-Regler oder ein PID-Regler sein.

Der Regler 5 gibt als Stellgröße eine bestimmte Spritzwassertemperatur $T^i_{set}$ aus, die einem weiteren Vergleicher 5a des ersten Regelkreises 1 zugeführt wird. In diesem weiteren Vergleicher 5a wird die vom Regler 5 ausgegebene Spritztemperatur mit der tatsächlichen Spritztemperatur $T^i_{Spritz}$ verglichen, wobei ein in Fig. 1 ebenfalls nicht bezeichnetes Ausgangssignals des Vergleichers 5a ausgegeben wird, das einer Regelabweichung zwischen der vom Regler 5 bestimmten Spritztemperatur $T^i_{set}$ und der tatsächlichen Spritztemperatur $T^i_{Spritz}$ entspricht.

Dem Vergleicher 5a des ersten Regelkreises 1 ist ein Folgeregler 6, beispielsweise ein PID-Regler, nachgeschaltet, der einen in Fig. 1 nicht gezeigten Stellantrieb mit einem entsprechenden Stellsignal beaufschlagt. Dieser Stellantrieb treibt ein Stellglied 7, beispielsweise ein Ventil, an, das die Spritztemperatur $T^i_{Spritz}$ in der Pasteurisieranlage regelt.

Wie vorstehend erläutert, wird die tatsächliche Spritztemperatur $T^i_{Spritz}$ im Vergleicher 5a rückgeführt. Durch die Serienschaltung des Reglers 5 und des Folgereglers 6 des ersten Regelkreises 1 ergibt sich eine Kaskadenregelung, die die Güte des ersten Regelkreises verbessert.

Die im Zusammenhang mit der Kaskadenregelung erfolgte Rückführung der Spritztemperatur $T^i_{Spritz}$ stellt nicht den ersten Regelkreis 1 dar, sondern ist ein dem ersten Regelkreis 1 untergeordneter Regelkreis.

Neben der Rückkopplung der tatsächlichen Spritztemperatur $T^i_{Spritz}$ wird aus der tatsächlichen Spritztemperatur $T^i_{Spritz}$ in der Einrichtung 10 ein Ist-Wert des ersten Kriteriums berechnet und als das Signal $CR^1_{ist}$ ausgegeben.

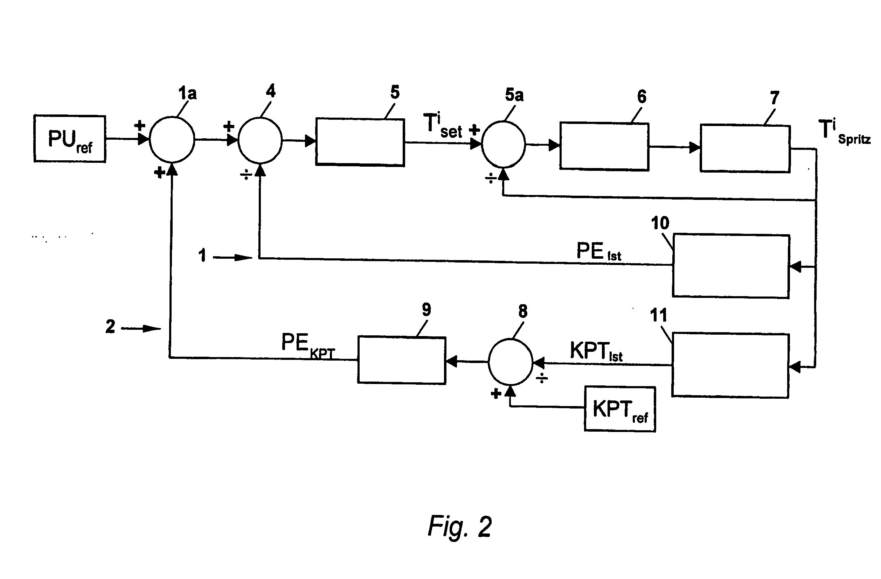

Wie im Zusammenhang mit der Beschreibung der Fig. 2 erläutert, ermittelt die Einrichtung 10 mit Hilfe eines computergestützten Rechenmodells die von einem Behälter in der Pasteurisieranlage aufgenommenen Pasteurisierungseinheiten, ausgehend von den vorgegebenen Spritztemperaturen $T^i_{Spritz}$ der jeweiligen Zonen. Dabei wird das Produkt mit der geringsten Anzahl an aufgenommenen Pasteurisierungseinheiten als Referenzprodukt ausgewählt, wobei der Pasteurisierungsgrad dieses Produktes dem Ist-Wert des ersten Kriteriums, also den übertragenen Pasteurisierungseinheiten, entspricht. Die Rückkopplung dieses Signals $CR^1_{ist}$ erfolgt über den Vergleicher 4, der, basierend auf dem Eingangssignal $CR^1_{ref}$, das den Soll-Wert des ersten Kriteriums, also den angestrebten Pasteurisierungsgrad entspricht, eine bestimmte Regelabweichung ausgibt.

Selbstverständlich ist die Erfindung nicht darauf beschränkt, dass die Bestimmung der aufgenommenen Pasteurisierungseinheiten als erstes Kriterium dient. Andere Kriterien, wie beispielsweise die Bestimmung von Oxidationseinheiten oder thermischen Zersetzungseinheiten, können ebenfalls als erstes Kriterium verwendet werden.

Der zweite Regelkreis 2, der dem ersten Regelkreis 1 überlagert ist, umfasst einen Vergleicher 8, wie in Fig. 1 gezeigt. In diesen Vergleicher 8 des zweiten Regelkreises 2 wird ein Eingangssignal $CR^2_{ref}$ eingespeist, das einem Soll-Wert des zweiten Regelkriteriums entspricht. Ferner wird in den Vergleicher 8 des zweiten Regelkreises 2 ein weiteres Signal $CR^2_{ist}$ eingespeist, das einem Ist-Wert des zweiten Regelkriteriums entspricht.

Aus diesen beiden Signalen $CR^2_{ref}$ und $CR^2_{ist}$ wird im Vergleicher 8 des zweiten Regelkreises eine Regelabweichung gebildet, die an den Regler 9 des zweiten Regelkreises 2 weitergeleitet wird. Im Regler 9 findet die Angleichung des Ist-Wertes an den Soll-Wert des zweiten Kriteriums statt, wobei als Stellgröße ein Ausgangssignal $CR^2_{set}$ vom Regler 9 des zweiten Regelkreises 2 ausgegeben wird.

Dieses Ausgangssignal $CR^2_{set}$, das die Regelabweichung zwischen dem Soll- und dem Ist-Wert des zweiten Regelkriteriums widerspiegelt, wird an der Mischstelle 1a in den ersten Regelkreis eingespeist. In der Mischstelle 1a wird also das Eingangssignal $CR^1_{ref}$ des ersten Regelkreises vom Ausgangssignal $CR^2_{set}$ des zweiten Regelkreises moduliert, so dass die im zweiten Regelkreis 2 stattgefundene Regelung des zweiten Kriteriums in die Regelung des ersten Regelkreises 1 eingeht.

Wird beispielsweise, wie im Zusammenhang mit Fig. 2 genauer erläutert, der erste Regelkreis 1 nach der Anzahl der aufgenommenen Pasteurisierungseinheiten und der zweite Regelkreis 2 nach der Abtöttemperatur geregelt, wird durch das Ausgangssignal $CR^2_{set}$ des Reglers 9 des zweiten Regelkreises 2 der Soll-Wert der Pasteurisierungseinheiten beispielsweise angehoben, wenn im Vergleicher 8 des zweiten Regelkreises festgestellt wird, dass die eingestellte Abtöttemperatur nicht erreicht wird.

Wie weiter in Fig. 1 gezeigt, bildet die Spritztemperatur $T^i_{Spitz}$ die Grundlage für die Berechnung des Ist-Wertes des ersten Kriteriums und des Ist-Wertes des zweiten Kriteriums. Die Berechnung der beiden Ist-Werte erfolgt in den Einrichtungen 10, 11 mit Hilfe eines computergestützten Rechenmodells, wobei die Einrichtung 10 beispielsweise die Anzahl der vom Produkt in der Pasteurisieranlage aufgenommenen Pasteurisierungseinheiten und die Einrichtung 11 die im Produkt herrschende Abtöttemperatur berechnet. Die Berechnung der jeweiligen Regelkriterien, also beispielsweise die Anzahl der übertragenen Pasteurisierungseinheiten, erfolgt auf an sich bekannte Weise und wird deshalb hier nicht mehr erläutert.

Die weiteren Regelkreise 3 bis N können wie der zweite Regelkreis 2 aufgebaut sein und verarbeiten eine Regelabweichung eines weiteren Regelkriteriums, das sich ebenfalls aus der Spritztemperatur durch Anwendung eines Rechenmodells ergibt.

Die Ausgangssignale $CR^3_{set}$ bis $CR^N_{set}$ der weiteren Regelsignale werden indirekt der Mischstelle 1a des ersten Regelkreises 1 zugeführt und beeinflussen dadurch das Eingangssignal $CR^1_{ref}$. Die indirekte Zuführung der jeweiligen Ausgangssignale $CR^3_{set}$ bis $CR^N_{set}$ zur Mischstelle 1a erfolgt über weitere Mischstellen 2a bis Na. Diese weiteren Mischstellen 2a bis Na verbinden jeweils einen vorangehenden Regelkreis mit einem darauffolgenden Regelkreis. Das bedeutet, dass das Ausgangssignal des nachfolgenden Regelkreises an der Mischstelle des vorangehenden Regelkreises das Ausgangssignal des vorangehenden Regelkreises moduliert, wobei das modulierte Ausgangssignal des vorangehenden Regelkreises der Mischstelle 1a bzw. der Mischstelle eines weiteren, ebenfalls vorangehenden Regelkreises zugeführt wird.

Selbstverständlich ist es auch möglich, die Zuführung der Ausgangssignale $CR^3_{set}$ bis $CR^N_{set}$ zur Mischstelle 1a des ersten Regelkreises anders zu gestalten. Es ist auch denkbar, die jeweiligen Ausgangssignale $CR^3_{set}$ bis $CR^N_{set}$

der weiteren Regelkreise 3 bis N direkt der Mischstelle 1 a des ersten Regelkreises 1 zuzuführen. Auch eine Kombination aus direkter und indirekter Zuführung der jeweiligen Ausgangssignale ist möglich. Die Anordnung mehrerer Mischstellen anstelle einer Mischstelle 1a des ersten Regelkreises 1 ist ebenfalls möglich, um das Eingangssignal $CR^1_{ref}$ mit Hilfe der jeweiligen Ausgangssignale der weiteren Regelkreise zu modulieren.

Nachfolgend wird mit Bezug auf Fig. 2 ein weiteres Ausgangsbeispiel der erfindungsgemäßen Vorrichtung erläutert, die eine Regeleinrichtung mit zwei Regelkreisen umfasst.

Die in Fig. 2 gezeigte Regeleinrichtung umfasst einen ersten Regelkreis 1 und einen zweiten Regelkreis 2, der dem ersten Regelkreis 1 überlagert ist. Der erste Regelkreis 1 umfasst den Vergleicher 4, dem der Regler 5, beispielsweise ein PI-Regler oder ein PID-Regler, nachgeschaltet ist. Zwischen dem Regler 5 und dem Folgeregler 6, der beispielsweise ein PID-Regler ist, ist ein weiterer Vergleicher 5a vorgesehen. Dem Folgeregler 6 ist das Stellglied 7, beispielsweise ein Ventil, nachgeschaltet. Die Rückkopplung des ersten Regelkreises 1 erfolgt einmal über den Vergleicher 5a, der zwischen dem Regler 5 und dem Folgeregler 6 angeordnet ist. Im Vergleicher 5a wird die tatsächliche Spritztemperatur $T^i_{Spritz}$ rückgekoppelt.

Aus der Spritztemperatur $T^i_{Spritz}$ werden in der Einrichtung 10 mit Hilfe eines entsprechenden Modells die von den Produkten bzw. von einem Referenzprodukt tatsächlich aufgenommenen Pasteurisierungseinheiten berechnet. Die Einrichtung 10 zur Berechnung des Ist-Wertes der Pasteurisierungseinheiten gibt ein Signal $PE_{ist}$ aus, das im Vergleicher 4 rückgekoppelt wird.

Der Vergleicher 4 vergleicht das Ausgangssignal $PE_{ist}$, also die tatsächlich aufgenommenen Pasteurisierungseinheiten, mit dem Eingangssignal $PE_{ref}$ des ersten Regelkreises 1, das dem Soll-Wert der Pasteurisierungseinheiten entspricht. Die im Vergleicher 4 festgestellte Regelabweichung zwischen Ist-Wert und Soll-Wert der Pasteurisierungseinheiten wird in den Regler 5 eingespeist, der eine bestimmte Spritztemperatur $T^i_{set}$ als Stelltemperatur ausgibt, die in den Vergleicher 5a eingegeben wird. Diese Spritztemperatur wird mit der tatsächlichen Spritztemperatur $T^i_{Spritz}$ rückgekoppelt, wobei der Folgeregler 6, ausgehend von dieser Rückkopplung, den Stellantrieb des Stellglied 7 mit einem entsprechenden Stellsignal beaufschlagt. Das Stellglied 7 beeinflusst dann die Spritztemperatur $T^i_{Spritz}$ in den jeweiligen Zonen der Pasteurisieranlage.

Auch in diesem Beispiel ist die Kaskadenregelung, die den Regler 5, den Vergleicher 5a und den Folgeregler 6 umfasst, dem ersten Regelkreis 1 untergeordnet und dient einer weiteren Verbesserung der Regelgüte.

Der zweite Regelkreis 2 umfasste einen Regler 9, einen dem Regler 9 vorgeschalteten Vergleicher 8 sowie eine Einrichtung 11 zur Berechnung des Ist-Wertes des zweiten Kriteriums. Als zweites Regelkriterium wird vorliegend die Abtöttemperatur verwendet. Ein Soll-Wert der Abtöttemperatur wird als Eingangssignal $KPT_{ref}$ in den Vergleicher 8 eingespeist und dort mit einem Ist-Wert der Abtöttemperatur, d.h. einem Ausgangssignal der Einrichtung zur Berechnung des Ist-Wertes der Abtöttemperatur verglichen. Die sich im Vergleicher 8 ergebenden Regelabweichung wird in den Regler 9 eingegeben, der dieses so genannte lokale Kriterium überwacht.

Der Regler 9 gibt als Stellgröße ein Signal $PE_{KPT}$ aus, das der Mischstelle 1 a zugeführt wird. In Abhängigkeit davon, ob die tatsächliche Produkttemperatur unterhalb oder oberhalb der Abtöttemperatur liegt, was im Vergleicher 8 festgestellt wird, wird durch das Ausgangssignal $PE_{KPT}$ des Reglers 9 des zweiten Regelkreises 2 das Eingangssignal $PE_{ref}$ des ersten Regelkreises 1 beeinflusst. Wenn also beispielsweise der Soll-Wert der Abtöttemperatur in den Behältern, die in der Pasteurisieranlage befindlich sind, nicht erreicht wird, wird die Anzahl der Pasteurisiereinheiten der Soll-Größe $PE_{ref}$ durch das Ausgangssignal $PE_{KPT}$ erhöht. Dadurch findet eine Anpassung der Pasteurisierungseinheiten an die Abtöttemperatur statt.

Mit Hilfe des vorliegenden Ausführungsbeispieles der erfindungsgemäßen Vorrichtung wird nicht nur nach der Anzahl der aufgenommenen Pasteurisierungseinheiten geregelt, sondern auch nach der Abtöttemperatur. Dadurch wird vermieden, dass eine zusätzliche Wärmebehandlung an den normalen Durchlauf durch die Pasteurisieranlage angeschlossen werden muss, wenn ein anderes Kriterium als die Anzahl der übertragenen Pasteurisierungseinheiten, beispielsweise die Abtöttemperatur, nicht erfüllt ist. Im Ausführungsbeispiel der Fig. 2 wird sowohl die Anzahl der übertragenen Pasteurisierungseinheiten als auch die Abtöttemperatur gleichzeitig bei der Regelung berücksichtigt, so dass mögliche Stillstandszeiten der Pasteurisieranlage durch nachgeschaltete Wärmebehandlungen unterdrückt werden.

**Patentansprüche**

1. Vorrichtung zur Pasteurisierung von Produkten, insbesondere befüllten Behältern, wie Flaschen oder Dosen, mit einer Regeleinrichtung, die einen ersten Regelkreis (1) umfasst, der nach einem ersten Kriterium regelbar ist wobei wenigstens ein zweiter Regelkreis (2) vorgesehen ist, der nach einem zweiten Kriterium regelbar ist, wobei der zweite Regelkreis dem ersten Regelkreis überlagert ist,
**dadurch gekennzeichnet, dass**
der zweite Regelkreis (2) mit dem ersten Regelkreis (1) an einer Mischstelle (1a) verknüpft ist, der ein Eingangssignal $CR^1_{ref}$ des ersten Regelkreises (1), das einem Sollwert des ersten Kriteriums entspricht, und ein Ausgangssignal

$CR^2_{set}$ des zweiten Regelkreises (2) zuführbar sind, wobei das Ausgangssignal $CR^2_{set}$ des zweiten Regelkreises (2) das Eingangssignal $CR^1_{ref}$ des ersten Regelkreises (1) moduliert.

**2.** Vorrichtung nach Anspruch 1, **dadurch gekennzeichnet, dass** der erste Regelkreis (1) einen Vergleicher (4) umfasst, der der Mischstelle (1a) nachgeordnet ist, wobei der Vergleicher (4) das in der Mischstelle (3) modulierte Eingangssignal $CR^1_{ref}$ und ein weiteres Signal $CR^1_{ist}$ vergleicht, das einem Ist-Wert des ersten Kriteriums entspricht, und ein Ausgangssignal ausgibt, das einer sich aus dem Soll/Ist-Vergleich ergebenden Regelabweichung entspricht.

**3.** Vorrichtung nach Anspruch 2, **dadurch gekennzeichnet, dass** dem Vergleicher (4) wenigstens ein Regler (5, 6) und ein Stellglied (7) nachgeordnet sind, wobei das der Regelabweichung entsprechende Ausgangssignal des Vergleichers (4) dem Regler (5, 6) zuführbar ist.

**4.** Vorrichtung nach wenigstens einem der Ansprüche 1 bis 3, **dadurch gekennzeichnet, dass** der zweite Regelkreis (2) einen Vergleicher (8) aufweist, der ein Eingangssignal $CR^2_{ref}$ das einem Sollwert des zweiten Kriteriums entspricht, und ein weiteres Signal $CR^2_{ist}$ vergleicht, das einem Ist-Wert des zweiten Kriteriums entspricht, und ein Ausgangssignal ausgibt, das einer sich aus dem Soll/Ist-Vergleich ergebenden Regelabweichung entspricht.

**5.** Vorrichtung nach Anspruch 4, **dadurch gekennzeichnet, dass** dem Vergleicher (8) des zweiten Regelkreises (2) ein Regler (9) nachgeordnet ist, wobei das der Regelabweichung entsprechende Ausgangssignal des Vergleichers (8) des zweiten Regelkreises (2) diesem Regler (4) zuführbar ist, der das Ausgangssignal $CR^2_{ist}$, ausgibt, das der Mischstelle (1a) zur Modulation des Eingangssignals $CR^1_{ref}$ des ersten Regelkreises (1) zuführbar ist.

**6.** Vorrichtung nach wenigstens einem der Ansprüche 1 bis 5, **dadurch gekennzeichnet, dass** der erste und der zweite Regelkreis (1, 2) jeweils eine Einrichtung (10, 11) zur Berechnung der Ist-Werte der jeweiligen Kriterien aus einem Prozessparameter, insbesondere der Spritztemperatur $T^i_{Spritz}$ umfassen.

**7.** Vorrichtung nach wenigstens einem der Ansprüche 1 bis 6, **dadurch gekennzeichnet, dass** das erste Kriterium Pasteurisiereinheiten PE und das zweite Kriterium eine andere Größe, insbesondere eine Abtöttemperatur KPT, umfassen.

**8.** Vorrichtung nach wenigstens einem der Ansprüche 1 bis 7, **dadurch gekennzeichnet, dass** weitere Regelkreise (3, ...N) vorgesehen sind, die jeweils nach weiteren Kriterien regelbar sind, wobei die Ausgangssignale $CR^3_{set}$ ...$CR^N_{set}$ der weiteren Regelkreise (3, ... N) der Mischstelle (1a) zuführbar sind und das Eingangssignal $CR^1_{ref}$ des ersten Regelkreises (1) beeinflussen.

**9.** Vorrichtung nach Anspruch 9, **dadurch gekennzeichnet, dass** die weiteren Regelkreise (3, ... N) jeweils eine Mischstelle (3a ... Na) umfassen, an der jeder weitere Regelkreis mit dem vorangegangen Regelkreis verknüpft ist.

**10.** Verfahren zum Pasteurisieren von Produkten, insbesondere befüllten Behältern, wie Flaschen oder Dosen, wobei ein erster Regelkreis (1) nach einem ersten Kriterium geregelt wird, wobei wenigstens ein zweiter Regelkreis (2) nach einem zweiten Kriterium geregelt wird, der dem ersten Regelkreis (1) überlagert ist,
**dadurch gekennzeichnet, dass**
der zweite Regelkreis (2) mit dem ersten Regelkreis (1) an einer Mischstelle (1 a) verknüpft ist, und ein Eingangssignal $CR^1_{ref}$ des ersten Regelkreises (1), das einem Sollwert des ersten Kriteriums entspricht, und ein Ausgangssignal $CR^2_{set}$ des zweiten Regelkreises (2) der Mischstelle zugeführt werden, wobei das Ausgangssignal $CR^2_{set}$ des zweiten Regelkreises (2) das Eingangssignal $CR^1_{ref}$ des ersten Regelkreises (1) moduliert wird.

### Claims

**1.** An apparatus for pasteurizing products, in particular filled receptacles, such as bottles or cans, said apparatus being provided with a control unit comprising a first control circuit (1) which is adapted to be controlled according to a first criterion, wherein at least one second control circuit (2) is provided, which is adapted to be controlled according to a second criterion, said second control circuit being superimposed on said first control circuit,
**characterized in that**
the second control circuit (2) is linked to the first control circuit (1) at a mixing point (1a), which is adapted to have supplied thereto an input signal $CR^1_{ref}$ of the first control circuit (1) that corresponds to a reference value of the first criterion, and an output signal $CR^2_{set}$ of the second control circuit (2), said output signal $CR^2_{set}$ of the second control circuit (2) modulating the input signal $CR^1_{ref}$ of the first control circuit (1).

2. An apparatus according to claim 1, **characterized in that** the first control circuit (1) comprises a comparator (4) arranged subsequent to the mixing point (1a), said comparator (4) comparing the input signal $CR^1_{ref}$ modulated in the mixing point (1a) and a further signal $CR^1_{measure}$ corresponding to an actual value of the first criterion, and outputting an output signal which corresponds to a control deviation resulting from the reference value/actual value comparison.

3. An apparatus according to claim 2, **characterized in that** the comparator (4) is followed by at least one controller (5, 6) and one actuator (7), the output signal of the comparator (4), which corresponds to the control deviation, being adapted to be supplied to the controller (5, 6).

4. An apparatus according to at least one of the claims 1 to 3, **characterized in that** the second control circuit (2) includes a comparator (8) which compares an input signal $CR^2_{ref}$ corresponding to a reference value of the second criterion and a further signal $CR^2_{measure}$ corresponding to an actual value of the second criterion, and which outputs an output signal corresponding to a control deviation resulting from the reference value/actual value comparison.

5. An apparatus according to claim 4, **characterized in that** the comparator (8) of the second control circuit (2) is followed by a controller (9), the output signal of the comparator (8) of the second control circuit (2), which corresponds to the control deviation, being adapted to be supplied to this controller (4) which outputs the output signal $CR^2_{measure}$ that is adapted to be supplied to the mixing point (1a) for modulating the input signal $CR^1_{ref}$ of the first control circuit (1).

6. An apparatus according to at least one of the claims 1 to 5, **characterized in that** the first and the second control circuit (1, 2) each comprise a unit (10, 11) for calculating the actual values of the respective criteria from a process parameter, in particular the spray temperature $T^i_{spray}$.

7. An apparatus according to at least one of the claims 1 to 6, **characterized in that** the first criterion comprises pasteurization units PU and the second criterion a different magnitude, in particular a lethal temperature KPT.

8. An apparatus according to at least one of the claims 1 to 7, **characterized in that** additional control circuits (3, ...N) are provided, which are each adapted to be controlled according to further criteria, the output signals $CR^3_{set}$ .... $CR^N_{set}$ of said additional control circuits (3, ... N) being adapted to be supplied to the mixing point (1a) and influencing the input signal $CR^1_{ref}$ of the first control circuit (1).

9. An apparatus according to claim 8, **characterized in that** the additional control circuits (3, .... N) each comprise a mixing point (3a ... Na) at which each additional control circuit is linked to the respective preceding control circuit.

10. A method of pasteurizing products, in particular filled receptacles, such as bottles or cans, wherein a first control circuit (1) is controlled according to a first criterion, wherein at least one second control circuit (2) is controlled according to a second criterion, said second control circuit (2) being superimposed on said first control circuit (1), **characterized in that** the second control circuit (2) is linked to the first control circuit (1) at a mixing point (1a), and that an input signal $CR^1_{ref}$ of the first control circuit (1), which corresponds to a reference value of the first criterion, and an output signal $CR^2_{set}$ of the second control circuit (2) are supplied to said mixing point, said output signal $CR^2_{set}$ of the second control circuit (2) modulating the input signal $CR^1_{ref}$ of the first control circuit (1).

**Revendications**

1. Dispositif de pasteurisation de produits, en particulier de récipients remplis, tels que bouteilles ou boîtes, comprenant un système de régulation qui comporte un premier circuit de régulation (1) pouvant être réglé suivant un premier critère, au moins un second circuit de régulation (2), réglable d'après un second critère, étant prévu, le second circuit de régulation étant superposé au premier circuit de régulation, **caractérisé en ce que** le second circuit de régulation (2) est combiné au premier circuit de régulation (1) en un point de mélange (1a), auquel peuvent être transmis un signal d'entrée $CR^1_{ref}$ du premier circuit de régulation (1), correspondant à une valeur de consigne du premier critère, et un signal de sortie $CR^2_{set}$ du second circuit de régulation (2), le signal de sortie $CR^2_{set}$ du second circuit de régulation (2) modulant le signal d'entrée $CR^1_{ref}$ du premier circuit de régulation (1).

**2.** Dispositif suivant la revendication 1, **caractérisé en ce que** le premier circuit de régulation (1) comprend un comparateur (4) monté en aval du point de mélange (1a), le comparateur (4) comparant le signal d'entrée $CR^1_{ref}$ modulé dans le point de mélange (1a) avec un autre signal $CR^1_{ist}$, qui correspond à une valeur effective du premier critère, et émettant un signal de sortie qui correspond à un écart de régulation résultant de la comparaison valeur de consigne/valeur effective.

**3.** Dispositif suivant la revendication 2, **caractérisé en ce qu'**au moins un régulateur (5, 6) et un organe de réglage (7) sont montés en aval du comparateur (4), le signal de sortie du comparateur (4), correspondant à l'écart de régulation, pouvant être transmis au régulateur (5, 6,).

**4.** Dispositif suivant l'une au moins des revendications 1 à 3, **caractérisé en ce que** le second circuit de régulation (2) comporte un comparateur (8) qui compare un signal d'entrée $CR^2_{ref}$, correspondant à une valeur de consigne du second critère, avec un autre signal $CR^2_{ist}$, correspondant à une valeur effective du second critère, et émet un signal de sortie qui correspond à un écart de régulation résultant de la comparaison valeur de consigne/valeur effective.

**5.** Dispositif suivant la revendication 4, **caractérisé en ce qu'un** régulateur (9) est monté en aval du comparateur (8) du second circuit de régulation (2), le signal de sortie du comparateur (8) du second circuit de régulation (2), correspondant à l'écart de régulation, pouvant être transmis à ce régulateur (4) qui émet le signal de sortie $CR^2_{ist}$ transmissible au point de mélange (1a) pour la modulation du signal d'entrée $CR^1_{ref}$ du premier circuit de régulation (1).

**6.** Dispositif suivant l'une au moins des revendications 1 à 5, **caractérisé en ce que** le premier et le second circuit de régulation (1, 2) comportent chacun un dispositif de calcul (10, 11) des valeurs effectives des critères respectifs à partir d'un paramètre de processus, en particulier de la température d'aspersion $T^i_{Spritz.}$

**7.** Dispositif suivant l'une au moins des revendications 1 à 6, **caractérisé en ce que** le premier critère comprend des unités de pasteurisation PE et le second critère une autre grandeur, en particulier une température létale KPT.

**8.** Dispositif suivant l'une au moins des revendications 1 à 7, **caractérisé en ce que** d'autre circuits de régulation (3, ... N) sont prévus, réglables chacun suivant d'autres critères, les signaux de sortie $CR^3_{set}$ ... $CR^N_{set}$ des autres circuits de régulation (3, ... N) pouvant être transmis au point de mélange (1a) et influant sur le signal d'entrée $CR^1_{ref}$ du premier circuit de régulation (1).

**9.** Dispositif suivant la revendication 9, **caractérisé en ce que** les autres circuits de régulation (3, ... N) comportent chacun un point de mélange (3a ... Na), sur lequel chaque autre circuit de régulation est combiné au circuit de régulation précédent.

**10.** Procédé de pasteurisation de produits, en particulier de récipients remplis, tels que bouteilles ou boîtes, dans lequel un premier circuit de régulation (1) est réglé suivant un premier critère, au moins un second circuit de régulation (2), superposé au premier circuit de régulation (1), est réglé suivant un second critère,
**caractérisé en ce que**
le second circuit de régulation (2) est combiné au premier circuit de régulation (1) en un point de mélange (1a), et un signal d'entrée $CR^1_{ref}$ du premier circuit de régulation (1), correspondant à une valeur de consigne du premier critère, et un signal de sortie $CR^2_{set}$ du second circuit de régulation (2) sont transmis au point de mélange, le signal de sortie $CR^2_{set}$ du second circuit de régulation (2) modulant le signal d'entrée $CR^1_{ref}$ du premier circuit de régulation (1).

Fig. 1

*Fig. 2*